Europäisches Patentamt

European Patent Office

Office européen des brevets

(11) Publication number: **0 548 376 A1**

# EUROPEAN PATENT APPLICATION
## published in accordance with Art. 158(3) EPC

(21) Application number: 92915918.4

(22) Date of filing: 16.07.92

(86) International application number:
PCT/JP92/00905

(87) International publication number:
WO 93/01825 (04.02.93 93/04)

(51) Int. Cl.5: **A61K 37/02**, A61K 31/725

(30) Priority: **19.07.91 JP 179739/91**

(43) Date of publication of application:
**30.06.93 Bulletin 93/26**

(84) Designated Contracting States:
**DE FR GB IT MC**

(71) Applicant: OTSUKA PHARMACEUTICAL CO., LTD.
9, Kandatsukasacho 2-chome
Chiyoda-ku Tokyo 101(JP)

(72) Inventor: KAWAI, Kazuyoshi Haitsu-Soreiyu 502
130-2-3, Aza Mizuhokaitaku Mizuho, Matsushigecho
Itano-gun Tokushima 771-02(JP)
Inventor: KONISHI, Yasue Dai-5 Ainichi-Haitsu 1F-102
68-1, Aza Maegawa, Okuno Aizumi-cho
Itano-gun Tokushima 771-12(JP)
Inventor: NAKAI, Satoru
67-4, Aza Minamikawamuko Hiroshima Matsushige-cho
Itano-gun Tokushima 771-02(JP)
Inventor: ADACHI, Masakazu
3493-9, Ishiharamachi
Takasaki-shi Gunma 370(JP)

(74) Representative: Hansen, Bernd, Dr.rer.nat. et al
Hoffmann, Eitle & Partner Patentanwälte
Arabellastrasse 4 Postfach 81 04 20
W-8000 München 81 (DE)

(54) ANTITUMOR DRUG.

(57) An antitumor drug containing a cytokine, particularly IL-1 or IL-2, and heparin as the active ingredients, and a method for treating tumors by administering a cytokine, particularly IL-1 or IL-2, and heparin.

TECHNICAL FIELD

The present invention relates to a combination drug of cytokine and heparin, which is of medicinal use, and more particularly to a combination drug of a cytokine, such as, for example, interleukin-1 or interleukin-2, and heparin.

BACKGROUND ART

It is known that stimulated lymphocytes, monocytes and macrophages produce a variety of biologically active proteinous factors which modulate or modify responses of the immune system. These proteinous factors associated with the defense and immunity of an organism have for some time been referred to collectively as cytokine [Balkwill, F. R., et al., Immunol. Today, 10, 299 (1989)].

It was then elucidated that the effects of these active factors are not limited to the immune system but extend to various functions of the living body and that they are deeply involved in embryogenesis, differentiation, homeostasis and pathophysiology, and many types of cytokine have so far been discovered.

While there is no established classification of cytokines as yet, they are generally classified into several groups on the basis of similarity in action. For example, there are known interferon (IFN), interleukin (IL), colony stimulating factor (CSF), tumor necrosis factor (TNF), T-cell growth factor, etc. and each of these groups of cytokines has been further ramified. Among cytokines, not a few have several actions in common. Thus, for example, IL-1, IL-2, IFN, TNF, etc. are all known to have pyrogenic and antitumor activities.

IL-1 (interleukin-1), a proteinous factor chiefly released from monocytes and macrophages, is a polypeptide in the molecular weight range of about 12000 to 18000 [S. B. Mizel, et al., Immunol. Rev., 63, 51-71 (1982)].

IL-1 acts on a variety of cells and exhibits a diversity of biological actions, thus playing critical roles in nearly all the biological reactions, such as immunity, inflammation, hematopoiesis, internal secretions, brain nerve functions and so on. IL-1 is classified according to the isoelectric point into two types, pI 5 and pI 7, and the former is called $\alpha$-type and the latter $\beta$-type [Oppenheim, J. J. et al., Immunol. Today, 7, 45 (1986): Dinarello, C. A., Adv. Immunol., 44, 153 (1988)]. While, within the same type, IL-1 has a high amino acid sequence homology of 60 to 70% even among different species of animals, its homology between $\alpha$- and $\beta$-types is low even within the same species of animal. Moreover, it has been reported that whether it is $\alpha$- or $\beta$-type, IL-1 binds to the same receptor on the cell membrane [Lowenthal, K., et al., J. Exp. Med., 164, 1060 (1986)]. It is also reported that IL-1 shows a direct growth inhibitory and cytocidal action on various tumor cells [Onozaki, K., et al., J. Immunol., 135, 3962 (1985)] and that IL-1 exerts an antitumor effect [Nakamura, S., et al., Jpn. J. Cancer Res. 77, 767 (1986), North, R. J., et al., J. Exp. Med., 168, 2031 (1988)].

Meanwhile, IL-2 (interleukin-2) was discovered by Morgan et al. in 1976 as a T-cell growth factor in culture supernatants of lectin-stimulated peripheral blood lymphocytes [Morgan, D. A., et al., Science, 193, 1007-1008 (1976)]. It has been shown that IL-2 is a substance consisting of 133 amino acids and having a molecular weight of 15420 and that it not only has T-cell growth stimulating activity but also promotes the mitosis of thymocytes, activates cytotoxic T-cells, induce differentiation of B-cells, activate NK-cells and induce LAK activity [Taniguchi, T. et al., Nature, 302, 305-310 (1983)].

Initially, from the expectation of its antitumoral activity, IL-2 was administered systemically or locally but the effect was neither so remarkable as expected nor commensurate with the consequent adverse reactions such as fever, general malaise, hypotension, eosinophilia and elevation of serum bilirubin.

However, it was discovered in 1982 that lymphocytes from cancer patients are activated, when cultured in the presence of IL-2, into lymphokine activated killer cells (LAK cells) which are highly cytotoxic even to autologous tumor cells [Grimm, E. A., et al., J. Exp. Med., 155, 1823 (1982)], and Rosenberg et al. reported that, following preclinical experiments, an IL-2-LAK cell combination therapy could be performed with success in malignant melanoma [Rosenberg, S. A., et al., N. Engl. J. Med., 313, 1485 (1985)]. This therapy is still attempted in the world even today but has not been found to be as effective as initially expected, either.

On the other hand, heparin is a strong organic acid having many sulfate groups and a potent anticoagulant as well. Heparin acts on thromboplastin to inhibit the conversion of prothrombin to thrombin and also inhibits the formation of fibrin from fibrinogen. As such, it has been used widely as an anticoagulant drug or a therapeutic/prophylactic drug for embolic thrombosis. It has also been reported that heparin has lipemic plasma clearing action, antihistaminic action, anticomplement action and tumor metastasis inhibitory action. The antitumor effect ascribable to the combined use of a heparin derivative and a corticoid steroid having antiinflammatory activity is described in Japanese Unexamined Patent Publication No. 60-115525. The tumor metastasis inhibitory activity of heparin is described in Japanese Unexamined Patent Publication No. 2-502006. Tari et al. also report that sustained intravenous injection of heparin is prophylactically effective against postoperative metastasis of cancer [Tari, Kiyonobu. et al., Journal of Clinical and Experimental Madicine 148 (1) 59-60 (1989)]. Sato et al. reported that neocarcinostatin, an anticancer agent, in a lipiodol-heparin emulsion produced anticancer and antitumor effects on rat ascite hepatoma cells and in liver cancer patient with human liver cirrhosis [Sato, Hiroshi. et al., Journal of Medicine and Pharmaceutical Science, 14 (2) 535-538 (1985); Japanese Unexamined Patent publication No. 3-14522]. Ataka reports that the suppression of the cytotoxicity of LAK cells by fibrin formation was inhibited by heparin in vitro [Ataka, S. et al., Journal of Clinical and Experimental Medicine, 155 (5) 351-352 (1990)]. However, there is no report indicating that heparin as it is has any direct antitumoral efficacy.

Both IL-1 and IL-2 inhibit the proliferation of tumor cells. However, in the clinical trial using an antitumor composition containing IL-1 as the active ingredient, side effects such as fever, gastrointestinal symptoms, general malaise, hypotension and myalgia were reported [H. A. Harvey, et al., Proc., ASCO., 24, 46 (1983)]. Thus, despite the expectations on IL-1 for cancer therapy, the idea of administration thereof in high doses for example is forced to be abandoned because of the aforesaid side effects.

Meanwhile, IL-2 as such is little effective and today a LAK therapy constitutes the main stream of therapy employing IL-2. However, this LAK therapy involves a time-consuming procedure prior to administration and is also disadvantageous in terms of equipment required for therapy. Thus, it is usually necessary to isolate, as the LAK cell starting material, the patient's own peripheral blood lymphocytes, lymphocytes infiltrating the tumor mass or lymphocytes in cancerous pleuritic ascites and incubate them together with IL-2 and this procedure requires a large amount of culture medium and a fairly large incubation space and, depending on kinds of materials, incubation time ranges from 1 to 2 weeks and the incubation must be continued for further 1 to 2 weeks before reaching the necessary cell population. Furthermore, the LAK therapy also involves adverse side effects such as fever, gastrointestinal symptoms, body weight gain due to body fluid retention, eosinophilia and anemia.

With regard to the application of heparin to cancer therapy, maintenance of LAK activity, inhibition of cancer metastasis, and combination therapy with an anticancer antibiotic have been reported but we do not know of a report suggesting its direct efficacy or on direct combination therapy with cytokine.

DISCLOSURE OF INVENTION

The inventors of the present invention surprisingly discovered, after intensive research to solve the above-mentioned problems, that the conjoint use of cytokine and heparin, particularly of IL-1 and heparin or of IL-2 and heparin, produces a tumor growth inhibitory effect even at a low dose at which IL-1 or IL-2, used alone, does not display its antitumor activity, thus permitting a drastic reduction in the dosage of IL-1 or IL-2. The present invention has been developed on the basis of the above finding.

The present invention provides an antitumor composition comprising a cytokine, particularly IL-1 or IL-2, and heparin as active ingredients together with a pharmaceutically acceptable carrier.

The present invention further provides a method of treating a cancer in a patient which comprises administering an effective amount of a cytokine having antitumor activity, particularly IL-1 or IL-2, and an effective amount of heparin to the patient with cancer in need of such therapy.

The present invention further provides the use of a combination of a cytokine having antitumor activity, particularly IL-1 or IL-2, and heparin for the production of an antitumor composition.

The cytokine, for example IL-1, which is used as an active ingredient in the present invention includes naturally occurring IL-1 and recombinantly produced IL-1 insofar as such have IL-1 activity. Moreover, IL-1 may be whichever of IL-1$\alpha$ and IL-1$\beta$, each of which may also be any of naturally occurring one and those produced by recombinant DNA technologies. Naturally occurring IL-1 can be produced by the method described in Japanese Unexamined Patent Publication No. 62-174022. Regarding the IL-1 obtainable by recombinant DNA technology, a variety of IL-1 derivatives can be obtained by the methods described in inter alia Japanese Unexamined Patent Publication No. 63-152398 and Japanese Unexamined Patent Publication No. 2-167298.

Among these IL-1, particularly IL-1$\alpha$ as such and IL-1$\beta$ as such, the IL-1$\beta$ derivatives and IL-1$\alpha$ derivatives described in Japanese Unexamined Patent Publication No. 63-152398 and No. 2-167298 referred to above, respectively, and the IL-1$\beta$ derivatives and IL-1$\alpha$ derivatives described in European Patent Applications publised under numbers 237073, 187991 and 237967 can be mentioned in the present invention.

As IL-1$\alpha$, the following may be mentioned as preferred examples.

    *  : Naturally occurring IL-1$\alpha$,

3

\* : IL-1α derivatives wherein at least one of Asn in 36-position and Cys in 141-position is(are) deficient or replaced by a different amino acid residue; preferably an IL-1α derivative having Asp in lieu of Asn in 36-position or an IL-1α derivative having Ser in lieu of Cys in 141-position;

\* : IL-1α derivatives wherein Arg in 16-position is deficient or substituted by a different amino acid residue, preferably an IL-1α derivative having Gly in lieu of Arg in 16-position;

\* : IL-1α derivatives each having a modified amino acid sequence meeting at least one of the conditions of being deficient in the amino acid sequence from Ser in 1-position to Phe in 14-position and being deficient in the amino acid sequence from Ser in 1-position to Met in 15-position.

Incidentally, the above description of IL-1α and IL-1α derivatives is based on the amino acid sequence disclosed in Japanese Unexamined Patent Publication No. 2-167298 referred to hereinbefore.

The IL-1β derivative which can be used as an active ingredient in the present invention includes the following as preferred examples.

\* : Naturally occurring IL-1β;

\* : an IL-1β derivative wherein Arg in 4-position is replaced by Gly;

\* : an IL-1β derivative wherein Cys in 8-position is replaced by Ala;

\* : an IL-1β derivative wherein Cys in 71-position is replaced by Ser;

\* : an IL-1β derivative wherein Cys in 71-position is replaced by Ala;

\* : an IL-1β derivative wherein Lys in 93-position is replaced by Leu;

\* : an IL-1β derivative wherein Trp in 120-position is replaced by Arg;

\* : an IL-1β derivative wherein His in 30-position is replaced by Tyr;

\* : IL-1β derivatives wherein Cys in 8-position and Cys in 71-position is replaced by Ala in 8-position and Ala in 71-position;

\* : IL-1β (4-153) polypeptide (an IL-1β derivative wherein the amino acid sequence from Ala in 1-position to Val in 3-position is deficient);

\* : IL-1β (1-150) polypeptide (an IL-1β derivative wherein the amino acid residues in 151-position et seq. are deficient)

The above description of IL-1β and IL-1β derivatives is based on the amino acid sequence disclosed in Japanese Unexamined Patent Publication No. 152398/1988 and European Patent Application publised under number 237967.

The IL-2 which can be used as the cytokine in accordance with the invention may be any substance having IL-2 activity, i.e., a capacity to maintain T-cells in culture. Thus, for example, naturally occurring IL-2 produced in animal bodies or cells, a recombinantly produced IL-2 and its analogues are included. The IL-2 and analogues mentioned above may or may not have sugar chains.

Naturally occurring IL-2 was first produced by culturing, for example, human peripheral lymphocytes or other IL-2-producing cell lines, and can be produced by reference to United States Patent No. 4401756. Recombinant IL-2 can be produced in accordance with the procedure described in Japanese Unexamined Patent Publication No. 61-78799.

Regarding the specific activity of IL-2, there is no established international unit, and any arbitrary unit system can be employed for IL-2. Thus, the IL-2 according to Japanese Unexamined Patent Publication No. 61-78799 is described to have a specific activity of about $3.5 \times 10^4$ units/mg referring to Japanese Unexamined Patent Publication No. 60-115528 for the method of specific activity determination and the definition of one unit. The specific activity of the IL-2 described in Japanese Unexamined Patent Publication No. 62-223129 is expressed in Jurkat units (JU) based on a BRM reference standard of Jurkat(human T-leukemia cell line)-derived IL-2 [Lot No. ISDP-841, National Cancer Institute (NCI)] and its specific activity mentioned is about $1.4 \times 10^7$ JU/mg.

Moreover, in his clinical study report on IL-2, Ohe states that the specific activities of the recombinant IL-2 used were $1 \times 10^7$ U/mg protein for S-6820 (Shionogi & Co., Ltd.) and $4.2 \times 10^4$ U/mg protein for TGP-3 (Takeda Chemical Industries, Ltd.). It is, thus, reported that the specific activity of TGP-3 is about 100-fold as high as the specific activity of NCI's IL-2 and the specific activity of S-6820 is about 2.5-fold higher as compared with NCI's. [Ohe Yuichiro & Saijo Nagahiro: Japanese Journal of Clinical Medicine 46 (5) 117-122 (1988)]. The maximum tolerable doses of the above IL-2 are reportedly $6.7 \times 10^5$ - $1.1 \times 10^6$ $\overline{U/m}^2$/day for S-6820 and, as determined in phase I study, 1000 U/body for TGP-3.

On the other hand, as the heparin comprising the other active ingredient in the antitumor composition of the invention, any commercial preparations of heparin sodium, heparin calcium, etc. which are available for medicinal use can be employed. Thus, for example, Heparin Sodium (Takeda-Shimizu), Novo Heparin (Kodama-Novo), Hepacarin (Eizai), etc. can be mentioned. Heparin sodium and heparin calcium are mucopolysaccharide sulfates having a molecular weight of about 6000 to 20000, and 1 ml - 100 ml injections containing 1000 - 5000 units per ml are already on the market and widely used as drugs.

Heparin sodium is listed in Japanese Pharmacopoeia X and the methods for production and assay of this salt are described in detail in THE MANUAL OF THE PHARMACOPOEIA OF JAPAN, TENTH EDITION (SOCIETY OF JAPANESE PHARMACOPOEIA, Hirokawa Publishing Co., 1981) at C-1380-1389.

As raw materials of heparin, extracts of bovine lung or porcine or sheep intestinal mucosa, among others, are employed.

One unit of heparin as used herein is defined by the unit described for heparin sodium in THE MANUAL OF THE PHARMACOPOEIA OF JAPAN, TENTH EDITION at C-1380 to C-1389.

The heparin which can be used in the present invention further includes heparin, N-desulfate heparin or N-acetylated heparin and other heprin derivatives.

In this specification and claims appended thereto, the term 'heparin' is used to mean, collectively, heparin salts such as said heparin sodium and heparin calcium, heparin and said heparin derivatives.

The antitumor composition of the present invention can be used in such a manner that cytokine and heparin, for example IL-1 and heparin or IL-2 and heparin, are provided in one dosage form and administered, or alternatively, cytokine and heparin, for example, either IL-1 and heparin or IL-2 and heparin, may be respectively provided in independent and separate dosage forms, which may be administered simultaneously or separately. In ether case, as will be apparent from the pharmacologic data to be described hereinafter, the cytokine and heparin, for example IL-1 and heparin or IL-2 and heparin, are considered to potentiate the tumor cell growth inhibitory activity of each other. Therefore, the proportions of the two ingredients can be selected from a very broad range.

In the clinical application of the antitumor composition of the present invention, the effective daily dosage for adult humans can be selected from a broard range. For example, IL-1 may be used in the daily dose range of generally about 0.01 $\mu$g/body to 100 $\mu$g/body and preferably about 0.1 $\mu$g/body to 10 $\mu$g/body. On the other hand, heparin may be used in the daily dose of generally about 10 units/body to 5 x $10^5$ units/body, particularly about 50 units/body to 5 x $10^5$ units/body, and preferably about 500 units/body to 5 x $10^4$ units/body.

In the present invention, IL-1 and heparin potentiate the tumor cell growth inhibitory action of each other as mentioned hereinbefore, with the result that when heparin is used at a clinical dosage commonly used in this field, for instance, the usual clinical dosage of IL-1 can be reduced to about 1/100 through 9/10.

As to IL-2, too, its daily clinical effective dosage for adult humans can be selected from a broad range just as mentioned for IL-1. Thus, IL-2 can be used in the daily dose range of generally about 0.028 $\mu$g/body to 280 $\mu$g/body and preferably 0.28 $\mu$g/body to 28 $\mu$g/body. On the other hand, heparin is used in the daily dose range of about 50 units/body to 5 x $10^5$ units/body and preferably about 500 units/body to 5 x $10^4$ units/body.

In the present invention, IL-2 and heparin potentiate the tumor cell growth inhibitory action of each other as mentioned hereinbefore, with the result that when heparin is used at a clinical dosage commonly used in this field, for instance, the usual clinical dosage of IL-2 can be reduced to about 1/100 through 9/10.

To obviate the aforementioned side effects, IL-1 and IL-2 are preferably administered at a lower dose within the above range in the practice of the invention. There is virtually no limitation on the dose ratio of IL-1 and heparin in the antitumor composition containing IL-1 and heparin as active ingredients insofar as they are used in the respective effective dose ranges mentioned above. Thus, the ratio can be selected from a broad range. Generally, however, the heparin (U/body)/IL-1 ($\mu$g/body) ratio is about 10 to 5,000,000, particulary about 50 to 5,000,000, preferably about 500 to 500,000 and more advantageously about 5,000 to 50,000. The dose ratio of IL-2 and heparin in the antitumor composition containing IL-2 and heparin as active ingredients can also be selected from a broad range substantially without limitations insofar as the two ingredients are used within the respective effective dose ranges mentioned hereinbefore. Generally, however, the heparin (U/body)/IL-2 ($\mu$g/body) ratio is about 2 to 200,000, preferably about 20 to 20,000 and more desirably about 200 to 2,000.

From the above points of view, the pharmaceutical composition of the present invention is preferably provided to insure the above dose ratio of active ingredients, irrespective of whether heparin and cytokine (IL-1, IL-2, etc.) are formulated in one dosage form or in independent and separate dosage forms. Taking the antitumor composition containing IL-1 and heparin as active ingredients as an example, the ratio of IL-1 and heparin can be suitably selected from a broad range without particular limitations. Generally, however, the heparin (U)/IL-1 ($\mu$g) ratio is about 10 to 5,000,000, preferably about 500 to 500,000 and, for still better results, about 5,000 to 50,000. The ratio of IL-2 and heparin in the antitumor composition containing IL-2 and heparin as active ingredients can also be selected from a broad range substantially without limitations. Generally speaking, heparin (U)/IL-2 ($\mu$g) ratio is about 2 to 200,000, preferably about 20 to 20,000 and, for still better results, about 200 to 2,000.

The antitumor composition of the present invention can be provided in a variety of dosage forms but typically in liquid dosage forms. In the antitumor composition containing IL-1 and hparin as active ingredients, IL-1 and heparin can be used in the aforementioned ratio and generally satisfactory results can be obtained when IL-1 is used in a concentration of 0.01 to 100 $\mu$g/ml, preferably about 0.1 to 10 $\mu$g/ml and heparin is used in a concentration of about 10 to 100,000 U/ml, preferably 100 to 10,000 U/ml. Similarly in the case of the antitumor composition containing IL-2 and heparin as active ingredients, IL-2 and heparin can be used in the aforementioned ratio and generally satisfactory results can be obtained when IL-2 is used in a concentration of about 0.028 to 280 $\mu$g/ml, preferably about 0.28 to 28 $\mu$g/ml, and heparin is used in a concentration of about 10 to 100,000 U/ml, preferably about 100 to 10,000 U/ml.

Depending on the intended use, the antitumor composition of the present invention can be administered in a variety of dosage forms which are conventionally used in the art. Particularly, in the present invention, by utilizing at least one member selected from the group consisting of human serum albumin, ordinary L-amino acids, sugars and surfactants, the stability of the active ingredients, particularly of cytokine, e.g. IL-1 and IL-2, can be enhanced.

As the amino acids mentioned above, those which are commonly employed in injectable preparations, such as glycine, cysteine, etc., can be employed.

The sugars are not particularly limited. Thus, for example, monosaccharides such as glucose, mannose, galactose, etc., sugar alcohols such as mannitol, inositol, xylitol, etc., disaccharides such as sucrose, maltose, lactose, etc. and polysaccharides such as dextran, hydroxypropylstarch, etc. can be employed. These sugars can be used alone or in combination. Among the above sugars, sucrose, maltose, mannitol, inositol and dextran are particularly preferred.

The surfactants are not particulary limited, either and both ionic and nonionic surfactants can be employed. Particularly preferred are such surfactants as polyoxyethylene glycol sorbitan alkyl esters, polyoxyethylene alkyl ethers, sorbitan monoacyl esters, fatty acid glycosides and so on.

The amount of said sugar to be added, in cases where any of IL-1 and its derivatives mentioned hereinbefore (all of which are collectively referred to as "IL-1 active substance") is employed, for instance, is not less than about 0.1 mg, preferably about 1 to 100 mg, per $\mu$g of the IL-1 active substance. The amount of said surfactant is not less than about 0.0001 mg, preferably about 0.001 to 0.1 mg, per $\mu$g of IL-1 active substance. The level of addition of human serum albumin is not less than about 0.001 mg, preferably about 0.01 to 10 mg, per $\mu$g of IL-1 active substance. The level of addition of amino acid is preferably about 0.001 to 10 mg (where two or more different amino acids are employed, the total amount thereof) per $\mu$g of IL-1 active substance.

When IL-2 is employed, the amount of said sugar is not less than about 0.01 mg, preferably about 0.1 to 10 mg, per mg of any of IL-2 and its derivatives mentioned hereinbefore (all of which are now referred to collectively as "IL-2 active substance"). The amount of said surfactant is not less than about 0.00001 mg, preferably about 0.0001 to 0.01 mg, per $\mu$g of IL-2 active substance. The amount of human serum albumin is not less than about 0.0001 mg, preferably about 0.001 to 1 mg, per $\mu$g of IL-2 active substance. The amount of said amino acid is preferably about 0.001 to 10 mg (where two or more different species are used, the total amount thereof) per $\mu$g of IL-2 active substance.

The antitumor composition of the present invention can be formulated in the same manner as other pharmaceutical preparations of this kind. Thus, other pharmacologically active ingredients and conventional pharmaceutical additives can be incorporated in appropriate proportions.

Particularly, as one of such other ingredients that can be incorporated in the antitumor composition of the present invention, a sulfur-containing reducing agent is preferred for further improving the stability of IL-1 active substance or IL-2 active substance. Preferred examples of such sulfur-containing reducing agent include relatively mild reducing agents such as cysteine, N-acetylhomocysteine, thioctic acid and thioglycolic acid, salts thereof, thioethanolamine, thioglycerol, sodium thiosulfate, thiolactic acid, dithiothreitol, glutathione, etc. These reducing agents can be used alone or in combination. While there is no particular limitation on the amount of such reducing agent, it can be used in an amount of not less than about 0.001 mg, preferably about 0.01 to 10 mg (where two or more species are used, the total amount thereof), per $\mu$g of said IL-1 or IL-2 active substance.

The antitumor agent according to the present invention is preferably made isotonic with a buffer solution to provide an isotonic preparation. The buffer solution which can be used for this purpose typically includes a variety of buffer solutions in the pH range of about 4 to 8, preferably about pH 5 to 6, such as citric acid-sodium citrate, citric acid-sodium phosphate, acetic acid-sodium acetate and citric acid-borax systems.

The antitumor composition of the present invention can be provided in various dosage forms by formulating a pharmacologically effective amount of IL-1 active substance or IL-2 active substance, for instance, with heparin and said additives. As carriers for such dosage forms, the conventional excipients and diluents such as fillers, thickeners, binders, humectants, disintegrators, etc. can be employed. The dosage form is not particularly limited so far as it contains the active ingredients in the effective form. Thus, it can be solid dosage forms such as tablets, powders, granules, pills, etc. or liquid dosage forms such as solutions, suspensions, emulsions and so on. It may also be a lyophilized preparation which can be reconstituted into a liquid form by addition of an appropriate vehicle prior to use. These dosage forms can each be manufactured by the established pharmaceutical procedure.

The pharmaceutical preparations thus manufactured can be administered by the routes suitable for the respective dosage forms. For example, injections may be administered intravenously, intramuscularly subcutaneously, intradermally or intraperitoneally, and solid dosage forms may be administered orally or rectally. The administration may be conducted in 3 to 4 divided doses a day.

The antitumor composition containing cytokine and heparin, more particularly IL-1 or IL-2 and heparin, as active ingredients is useful for the treatment of various tumors, particularly the treatment of malignant tumors, such as melanoma, ovarian cancer, colon cancer, liver cancer, brain tumor, leukemia and so on.

By using cytokine and heparin in combination, particularly IL-1 and heparin or IL-2 and heparin in combination, in accordance with the present invention, the antitumor activity of each active substance can be remarkably potentiated so that a tumor cell growth inhibitory effect can be obtained even at a low dose where neither of the drugs, used alone, exhibit the desired antitumor effect. Consequently, there can be realized a drastic reduction in the dosage of IL-1 or IL-2 as well as mitigation of the adverse side effects mentioned hereinbefore.

## Examples

The following reference examples, pharmacological test examples and formulation examples are intended to illustrate the invention in further detail.

### Reference Example 1

An interleukin-1$\beta$ derivative wherein Cys in 71 position was replaced by Ser was synthesized and purified by the method described in Example 1 of Japanese Unexamined Patent Publication No. 63-152398.

### Pharmacological Test Example 1

The antitumor effect of the combined administration of the interleukin-1$\beta$ derivative obtained in Reference Example 1 (referred to briefly as "IL-1$\beta$" in this pharmaceutical test example) and heparin was evaluated in terms of growth inhibitory activity against A375S2 cells (human melanoma cell) in vitro.

The growth inhibitory activity against A375S2 cells (human melanoma cell) was assayed by the method of Nakai [Biochem. Biophys. Res. Commun., 154, 1189, (1988)]. Thus, cells obtained by subculture were washed twice with PBS (-) solution (Nissui Pharmaceutical) and detached with 0.05% trypsin (Flow Laboratories). The cells were pipetted and suspended in a culture medium composed of E-MEM (Eagles' Minimum Essential Medium, Nissui Pharmaceutical) and 10% FBS (fetal bovine serum, GIBCO). The cells were centrifugally washed (Hitachi, Ltd., 05PR-22) at 25°C and 1200 rpm for 5 minutes and, then, resuspended in the same medium as above. For the determination of viable cells, the cells were stained with trypan blue solution (Wako Pure Chemical) and observed under a light microscope (Olympus Optical, BH-2). The suspension was then diluted to $2 \times 10^4$ cells/ml.

The wells of a 96-well microplate (Corning) were filled with 0.1 ml/well of culture medium (E-MEM + 10% FBS) containing heparin (heparin sodium, manufactured by Novo) at a final concentration of 0, 20 or 40 U/ml. Then, 0.1 ml aliquots of IL-1$\beta$ having a concentration of 1 ng/ml were added to row A of the microplate and doubling dilution was repeated to row G. To each of the wells of the microplate carrying this dilution series was added 0.1 ml of an A375S2 cell suspension adjusted with the same culture medium to $2 \times 10^4$ cells/ml. This microplate was incubated at 37°C and under 5% $CO_2$ for 4 days (Napco $CO_2$ incubator).

Thereafter, 0.1 ml/well of E-MEM medium containing 0.05% of neutral red (Merck) was added to each well, and incubation was further continued at 37°C and under 5% $CO_2$ for additional 2 hours. The medium was then discarded and the wells were washed with PBS (-) solution. Then, 0.1 ml/well of eluent (0.1 M $NaH_2PO_4$ buffer + 50% ethanol, Wako Pure Chemical) was added to each well and after stirring with a micromixer (MX-4, Sankyo Pure Chemical), the absorbance O.D. at 540 nm was measured with an absorptiometer (Titertek Multiskan MMC, Flow Laboratories). The amount of the pigment taken up into the viable cells was determined from the absorbance and the cell growth inhibition rate (%) was calculated by means of the following equation.

Cell growth inhibition rate (%) = (1-M/C) x 100

[where M means the amount of pigment taken up into cells in the treatment group and C means the amount of pigment taken up in cells in the control group].

Incidentally, the growth inhibition rate of heparin as used alone was 10.0±1.5% at 20 U/ml and 22.3±3.7% at 40 U/ml. The test of significance of group-to-group differences was made by Student's t-test using as control a group wherein IL-1$\beta$ was used singly. The results are set forth in Table 1.

Table 1 Growth Inhibitory Activity (growth inhibition rate) against A375S2 Cells

| Test group | Dilution factor | | | | | | |
|---|---|---|---|---|---|---|---|
| | 4 | 8 | 16 | 32 | 64 | 128 | 256 |
| IL-1β (0.1 ng/ml) | 88.9 ±2.0 | 54.5 ±3.1 | 26.2 ±3.8 | 15.2 ±3.5 | 5.8 ±3.3 | 2.8 ±1.0 | 5.5 ±1.1 |
| IL-1β (0.1 ng/ml) + heparin (20 U/ml) | 86.3 ±1.6 | 59.1 ±4.4 | 40.5 ±6.3 | 41.6* ±7.4 | 23.4* ±5.0 | 16.5* ±5.5 | - |
| IL-1β (0.1 ng/ml) + heparin (40 U/ml) | 84.5 ±4.0 | 77.9** ±2.2 | 64.9** ±3.8 | 65.4** ±3.9 | 56.2** ±3.8 | 50.0** ±1.9 | 41.0** ±7.1 |

*: $p < 0.05$   **: $p < 0.01$

It is apparent from Table 1 that compared with IL-1$\beta$ single use group, a significant potentiation of growth inhibitory activity against A375S2 cells was obtained in the group treated with a combination of IL-1$\beta$ and either 20 or 40 U/ml of heparin.

Pharmaceutical Test Example 2

The antitumor effect of the combined administration of the interleukin-1$\beta$ derivative obtained in Reference Example 1 (referred to briefly as "IL-1$\beta$" in this pharmaceutical test example) and heparin was evaluated in terms of growth inhibitory activity against NIH:OVCAR-3 cells (human ovarian cancer cell) in vitro.

The growth inhibitory activity against NIH:OVCAR-3 cells (human ovarian cancer cell) was assayed by the method of Nakai [Biochem. Biophys. Res. Commun., 154, 1189, (1988)]. Thus, cells obtained by subculture were washed twice with PBS (-) solution (Nissui Pharmaceutical) and detached with 0.05% trypsin (Flow Laboratories). The cells were pipetted and suspended in a culture medium composed of RPMI-1640 medium (Gibco), 10% FBS (fetal bovine serum, Gibco) and 10 $\mu$g/ml insulin (Sigma). The cells were centrifugally washed (Hitachi, Ltd., 05PR-22) at 25°C and 1200 rpm for 5 minutes and, then, resuspended in the same medium. For the determination of viable cells, the cells were stained with trypan blue solution (Wako Pure Chemical) and observed under a light microscope (Olympus Optical, BH-2). The suspension was then diluted to $8 \times 10^4$ cells/ml.

The wells of a 96-well microplate (Corning) were filled with 0.1 ml/well of culture medium (RPMI-1640 + 10% FBS + 10 $\mu$g/ml insulin (Sigma)) containing heparin (heparin sodium, manufactured by Novo) at a final concentration of 0, 20 or 40 U/ml. Then, 0.1 ml aliquots of IL-1$\beta$ having a concentration of 1 ng/ml were added to row A of the 96-well microplate and doubling dilution was repeated to row H. To each of the wells of the 96-well microplate carrying this dilution series was added 0.1 ml of an NIH:OVCAR-3 cell suspension adjusted with culture medium to $8 \times 10^4$ cells/ml. This microplate was incubated (Napco $CO_2$ incubator) at 37°C and under 5% $CO_2$ for 4 days.

Thereafter, 0.1 ml/well of E-MEM medium containing 0.05% neutral red (Merck) was added to each well, and incubation was further continued at 37°C and under 5% $CO_2$ for additional 2 hours. The medium was then discarded and the wells were washed with PBS (-) solution. Then, 0.1 ml/well of eluent (0.1 M $NaH_2PO_4$ buffer + 50% ethanol, Wako Pure Chemical) was added to each well and after stirring with a micromixer (MX-4, Sankyo Pure Chemical), the absorbance O.D. at 540 nm was measured with an absorptiometer (Titertek Multiskan MMC, Flow Laboratories). The amount of the pigment taken up into the viable cells was determined from the absorbance value and the cell growth inhibition rate (%) was calculated by means of the following equation.

Cell growth inhibition rate (%) = (1-M/C) x 100

[where M means the amount of pigment taken up into cells in the treatment group and C means the amount of pigment taken up in cells in the control group].

Incidentally, the growth inhibition rate of heparin as used alone was 22.4±2.4% at 20 U/ml and 37.9±2.8% at 40 U/ml. The test of significance of group-to-group differences was made by Student's t-test using the IL-1$\beta$ single use group as control. The results are set forth in Table 2.

Table 2   Growth Inhibitory Activity (growth inhibition rate) against NIH: OVCAR-3 Cells

| Test group | Dilution factor | | | | | | | |
|---|---|---|---|---|---|---|---|---|
| | 4 | 8 | 16 | 32 | 64 | 128 | 256 | 512 |
| IL-1β (1 ng/ml) | 62.1 ±0.5 | 60.4 ±2.2 | 62.0 ±0.8 | 60.7 ±1.2 | 46.4 ±0.9 | 34.3 ±1.5 | 19.2 ±3.4 | 9.5 ±2.9 |
| IL-1β (1 ng/ml) + heparin (20 U/ml) | 65.1 ±2.5 | 68.8* ±1.4 | 72.1* ±2.7 | 70.5 ±3.4 | 67.9** ±2.7 | 58.6** ±3.3 | 41.3* ±3.9 | 39.5** ±3.4 |
| IL-1β (1 ng/ml) + heparin (40 U/ml) | 73.3** ±1.9 | 82.5** ±1.7 | 85.3*** ±1.0 | 86.9*** ±1.1 | 84.9*** ±0.9 | 80.8*** ±2.0 | 70.2*** ±2.0 | 64.5*** ±2.6 |

*: $p < 0.05$   **: $p < 0.01$   ***$p < 0.001$

It is apparent from Table 2 that compared with the IL-1β single use group, a significant potentiation of growth inhibitory activity against NIH:OVCAR-3 cells was obtained in the group treated with a combination of IL-1β and either 20 or 40 U/ml of heparin.

Pharmaceutical Test Example 3

The antitumor effects of the IL-1$\beta$ derivative (referred to briefly as "IL-1$\beta$" in this pharmaceutical test example) and heparin, used singly or in combination, were evaluated. The following method was used for determination.

Meth A sarcoma cells subcultured in the ascites of the female BALB/c mice (average body weight: 20 g) purchased from Japan SLC, Inc. were suspended in a 1:1 mixture of Hanks' solution (Flow Laboratories) and PBS (-) solution (Nissui Pharmaceutical). The suspension was centrifugally washed twice (Hitachi, Ltd., 05PR-22) at 1200 rpm (4°C) for 5 minutes and, then, resuspended in the same mixture as above. For the determination of viable cells, the cells were stained with trypan blue solution (Wako Pure Chemical) and observed under a light microscope (Olympus Optical, BH-2). The suspension was then diluted to 1 x 10$^6$ cells/ml. Then, using an injection syringe (Terumo, injection needle 26 G), 1 x 10$^5$ cells/0.1 ml/body were intradermally inoculated at the right abdominal region of mice. After 5 days, the long and short diameters of the tumor were measured with electric calipers (NSK, MAX-CAL) and the tumor weight was calculated by means of the following equation.

Tumor weight = long diameter x (short diameter)$^2$ x 1/2

The test mice were allocated to treatment groups with tumor weights aligned to the range of 100 to 110 mg. The test was performed in groups of 6 mice. A mixture of 1 $\mu$g/body of IL-1$\beta$ and 20 U/body of heparin (Novo, heparin sodium) was then administered, in 50 $\mu$l portions, into the tumors for 2 consecutive days from day 7 after the tumor transplantation. As the control group, 50 $\mu$l portions of PBS (-) containing 30 $\mu$g/ml mouse serum albumin (Boehringer Manheim-Yamanouchi), which was used for dissolving the test drugs, was administered into the tumor. For the mice in each test group, the long and short diameters of the tumor was serially measured to calculate the tumor weight from day 6 after tumor transplantation. The test of significance of group-to-group differences was made by Student's t-test using as control a group to which solvent was administered. The results are set forth in Table 3.

12

Table 3

| Test gruop | Days after tumor transplantation | | | | | |
|---|---|---|---|---|---|---|
| | 6 | 11 | 16 | 21 | 28 | 31 |
| Control (solvent) | 109±10 | 260±35 | 458±63 | 880±144 | 2652±409 | 3617±535 |
| IL-1β 1 μg/body | 108±12 | 226±26 | 316±75 | 649±231 | 2321±847 | 3162±1173 |
| Heparin 20 U/body | 106±11 | 280±17 | 382±31 | 865±63 | 2412±233 | 2948±273 |
| IL-1β 1μg/body + heparin 20 U/body | 105±9 | 196±28 | 240±26** | 339±107* | 1181±467* | 1659±644* |

*: $p < 0.05$     **: $p < 0.01$

An antitumor effect, though weak, was found in the group treated with 1 $\mu$g/body of IL-1$\beta$. On the other hand, a clearly significant antitumor effect was found in the group treated with 1 $\mu$g/body of IL-1$\beta$ and 20 U/body of heparin, indicating that the combination treatment with IL-1$\beta$ and heparin results in potentiated antitumor efficacy.

Some formulation examples of the antitumor agent of the invention are given below.

Formulation Example 1

| Interleukin-1$\beta$ | 0.8 $\mu$g/ml |
|---|---|
| Heparin | 5000U/ml |
| Tween 80 | 0.01 mg/ml |
| Dextran | 15 mg/ml |
| Cysteine | 0.1 mg/ml |
| HSA (human serum albumin) | 1.0 mg/ml |

The above ingredients were added to 0.01 M citric acid-sodium citrate buffer (pH 6.0) at the above final concentrations and after stirring, the mixture was filtered (through a 0.22 $\mu$m membrane filter). The filtrate was aseptically distributed, in 1 ml portions, into vials and lyophilized to provide an antitumor composition of the invention in the form of injection. This preparation is used after dilution with 1 ml of physiological saline just prior to use.

Formulation Example 2

| Interleukin-1$\beta$ | 0.2 $\mu$g/ml |
|---|---|
| Heparin | 1000U/ml |
| Tween 80 | 0.01 mg/ml |
| Dextran | 15 mg/ml |
| Cysteine | 0.1 mg/ml |
| HSA (human serum albumin) | 1.0 mg/ml |

The above ingredients were added to 0.01 M citric acid-sodium citrate buffer (pH 6.0) at the above final concentrations and after stirring, the mixture was filtered (through a 0.22 $\mu$m membrane filter). The filtrate was aseptically distributed, in 1 ml portions, into vials and lyophilized to provide an antitumor composition of the invention in the form of infection. This preparation is used after dilution with 1 ml of physiological saline just prior to use.

Formulation Example 3

| Interleukin-2 | 2.8 $\mu$g/ml |
|---|---|
| Heparin | 5000U/ml |
| Tween 80 | 0.01 mg/ml |
| Dextran | 15 mg/ml |
| Cysteine | 0.1 mg/ml |
| HSA (human serum albumin) | 1.0 mg/ml |

The above ingredients were added to 0.01 M citric acid-sodium citrate buffer (pH 6.0) at the above final concentrations and after stirring, the mixture was filtered (through a 0.22 $\mu$m membrane filter). The filtrate was aseptically distributed, in 1 ml portions, into vials and lyophilized to provide an antitumor composition of the invention in the form of injection. This preparation is used after extemporaneous dilution with 1 ml of physiological saline.

Formulation Example 4

| Interleukin-2 | 0.56 $\mu$g/ml |
|---|---|
| Heparin | 1000U/ml |
| Tween 80 | 0.005 g/ml |
| Dextran | 5 mg/ml |
| Cysteine | 0.1 mg/ml |
| HSA (human serum albumin) | 0.5 mg/ml |

The above ingredients were added to 0.01 M citric acid-sodium citrate buffer (pH 6.0) at the above final concentrations and after stirring, the mixture was filtered (through a 0.22 $\mu$m membrane filter). The filtrate was aseptically distributed, in 1 ml portions, into vials and lyophilized to provide an antitumor composition of the invention in the form of injection. This preparation is used after dilution with 1 ml of physiological saline just prior to use.

**Claims**

1. An antitumor composition comprising cytokine and heparin as active ingredients together with a pharmaceutically acceptable carrier.

2. The antitumor composition according to claim 1 wherein the cytokine is naturally occurring or a recombinantly produced interleukin-1.

3. The antitumor composition according to claim 1 wherein the cytokine is naturally occurring interleukin-1$\beta$ or a recombinantly produced interleukin-1$\beta$ or interleukin-1$\beta$ derivative having interleukin-1$\beta$ activity.

4. The antitumor composition according to claim 1 wherein the cytokine is interleukin-2.

5. The antitumor composition according to claim 1 wherein the cytokine is naturally occurring interleukin-2 or a recombinantly produced interleukin-2 or an anologue thereof.

6. The antitumor composition according to claim 1 wherein the heparin is selected from the group consisting of heparin sodium, heparin calcium, heparin, N-desulfate heparin and N-acetylated heparin.

7. The antitumor composition according to claim 1 containing cytokine and heparin in a single dosage form.

8. The antitumor composition of claim 1 containing cytokine and heparin in two separate dosage forms.

9. The antitumor composition according to claim 1 which further contains at least one member selected from the group consisting of human serum albumin, L-amino acids, sugars and surfactants.

10. A method of treating a tumor which comprises administering a therapeutically effective amount of cytokine and a therapeutically effective amount of heparin to a cancer patient.

11. The method according to claim 10 wherein the cytokine is naturally occurring or a recombinantly produced interleukin-1.

12. The method according to claim 10 wherein the cytokine is naturally occurring interleukin-1$\beta$ or a recombinantly produced interleukin-1$\beta$ or interleukin-1$\beta$ derivative having interleukin-1$\beta$ activity.

13. The method according to claim 10 wherein the cytokine is interleukin-2.

14. The method according to in claim 10 wherein the cytokine is naturally occurring interleukin-2 or a recombinantly produced interleukin-2 or an analogue thereof.

**15.** The method according to claim 10 wherein the heparin is selected from the group consisting of heparin sodium, heparin calcium, heparin, N-desulfate heparin and N-acetylated heparin.

**16.** The method according to claim 10 wherein the cytokine and heparin are administered in a single dosage form.

**17.** The method according to claim 10 wherein the cytokine and heparin are administered in two separate dosage forms.

**18.** Use of a combination of cytokine and heparin for the production of an antitumor composition.

# INTERNATIONAL SEARCH REPORT

International Application No PCT/JP92/00905

## I. CLASSIFICATION OF SUBJECT MATTER (if several classification symbols apply, indicate all) [6]

According to International Patent Classification (IPC) or to both National Classification and IPC

Int. Cl$^5$ A61K37/02, 31/725

## II. FIELDS SEARCHED

### Minimum Documentation Searched [7]

| Classification System | Classification Symbols |
|---|---|
| IPC | A61K37/02, 31/725 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| X | JP, A, 2-9391 (The Rockenfeller University), January 12, 1990 (12. 01. 90), & EP, A, 310136 & AU, A, 8823336 | 1-7, 18 |
| Y | EP, A, 332952 (BASF A.G.), September 20, 1989 (20. 09. 89), & DE, A, 3808353 & AU, A, 8931221 & JP, A, 1-275532 | 1-7, 18 |
| Y | JP, A, 58-92620 (Sunstar Inc., and another), June 2, 1983 (02. 06. 83), & EP, A, 80879 & US, A, 4675184 & JP, B, 3-8323 | 1, 6, 7, 18 |
| A | JP, A, 3-127740 (Teijin Ltd.), May 30, 1991 (30. 05. 91), (Family: none) | 1, 7, 18 |
| A | JP, A, 2-111726 (Dainippon Pharmaceutical Co., Ltd.), April 24, 1990 (24. 04. 90), | 1-3, 7, 18 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| November 6, 1992 (06. 11. 92) | November 24, 1992 (24. 11. 92) |

| International Searching Authority | Signature of Authorized Officer |
|---|---|
| Japanese Patent Office | |

Form PCT/ISA/210 (second sheet) (January 1985)

International Application No. PCT/JP92/00905

| | FURTHER INFORMATION CONTINUED FROM THE SECOND SHEET | |
|---|---|---|
| | (Family: none) | |
| A | JP, A, 2-213 (Taiho Pharmaceutical Co., Ltd.), January 5, 1990 (05. 01. 90), (Family: none) | 1-5, 7, 18 |
| A | JP, A, 2-40399 (Takeda Chemical Industries, Ltd.), February 9, 1990 (09. 02. 90), (Family: none) | 1, 6, 7, 18 |
| A | JP, A, 59-176213 (President and Fellows of Harvard Callege), | 1, 6, 7, 18 |

**V.[X] OBSERVATIONS WHERE CERTAIN CLAIMS WERE FOUND UNSEARCHABLE [1]**

This international search report has not been established in respect of certain claims under Article 17(2) (a) for the following reasons:

1.[X] Claim numbers 10-17 because they relate to subject matter not required to be searched by this Authority, namely:

Claims 10 to 17 pertain to a medical treatment of the human or animal body by curing.

2.[ ] Claim numbers        , because they relate to parts of the international application that do not comply with the prescribed requirements to such an extent that no meaningful international search can be carried out, specifically:

3.[ ] Claim numbers        , because they are dependent claims and are not drafted in accordance with the second and third sentences of PCT Rule 6.4(a).

**VI.[ ] OBSERVATIONS WHERE UNITY OF INVENTION IS LACKING [2]**

This International Searching Authority found multiple inventions in this international application as follows:

1.[ ] As all required additional search fees were timely paid by the applicant, this international search report covers all searchable claims of the international application.

2.[ ] As only some of the required additional search fees were timely paid by the applicant, this international search report covers only those claims of the international application for which fees were paid, specifically claims:

3.[ ] No required additional search fees were timely paid by the applicant. Consequently, this international search report is restricted to the invention first mentioned in the claims; it is covered by claim numbers:

4.[ ] As all searchable claims could be searched without effort justifying an additional fee, the International Searching Authority did not invite payment of any additional fee.

Remark on Protest

[ ] The additional search fees were accompanied by applicant's protest

[ ] No protest accompanied the payment of additional search fees

Form PCT/ISA/210 (supplemental sheet (2)) (January 1985)

FURTHER INFORMATION CONTINUED FROM THE SECOND SHEET

October 5, 1984 (05. 10. 84),
& EP, A, 114589 & AU, A, 8322582
& CA, A, 1226816

---

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers      , because they relate to subject matter not required to be searched by this Authority, namely:

2.☐ Claim numbers      , because they relate to parts of the international application that do not comply with the prescribed requirements to such an extent that no meaningful international search can be carried out, specifically:

3.☐ Claim numbers      , because they are dependent claims and are not drafted in accordance with the second and third sentences of PCT Rule 6.4(a).

---

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest

☐ No protest accompanied the payment of additional search fees.

Form PCT/ISA/210 (supplemental sheet (2)) (January 1985)